Europäisches Patentamt

European Patent Office

Office européen des brevets

(11) Publication number: **0 167 662**
**B1**

(12) **EUROPEAN PATENT SPECIFICATION**

(45) Date of publication of patent specification: **19.12.90**

(21) Application number: **84111879.7**

(22) Date of filing: **04.10.84**

(51) Int. Cl.⁵: **A 61 M 5/178**, A 61 M 5/315,
A 61 M 5/32, A 61 M 5/00,
A 61 M 5/28

(54) **Multi-needle syringe for filling through a barrel plunger, particularly useful in mesotherapy.**

(30) Priority: **10.10.83 IT 1201883**

(43) Date of publication of application:
**15.01.86 Bulletin 86/03**

(45) Publication of the grant of the patent:
**19.12.90 Bulletin 90/51**

(84) Designated Contracting States:
**BE CH DE FR GB LI NL**

(56) References cited:
**CH-A- 547 099**
**DE-A-3 035 009**
**FR-A-1 104 570**
**GB-A-1 197 987**
**US-A-3 595 231**

(73) Proprietor: **Aluigi, Mario**
**Via San Paolo 18**
**I-47037 Rimini (Prov. of Forli) (IT)**
(73) Proprietor: **Mosconi, Paolo**
**Via Dardanelli 17**
**I-47037 Rimini (Prov. of Forli) (IT)**
(73) Proprietor: **Ricciotti, Pier Carlo**
**Via Mentana 36**
**I-47037 Rimini (Prov. of Forli) (IT)**

(72) Inventor: **Aluigi, Mario**
**Via San Paolo 18**
**I-47037 Rimini (Prov. of Forli) (IT)**
Inventor: **Mosconi, Paolo**
**Via Dardanelli 17**
**I-47037 Rimini (Prov. of Forli) (IT)**
Inventor: **Ricciotti, Pier Carlo**
**Via Mentana 36**
**I-47037 Rimini (Prov. of Forli) (IT)**

(74) Representative: **Modiano, Guido et al**
**MODIANO, JOSIF, PISANTY & STAUB Modiano**
**& Associati Via Meravigli, 16**
**I-20123 Milano (IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

## Description

In mesotherapeutical treatments, in order to obtain biological responses or desensitising effects, infradermal injections of specific medicinal mixtures are applied which, for their improved distribution through the derma depth, are split into small doses by means of syringes having special multi-needle offtakes.

Such offtakes comprise a cylindrical body of metal construction throughout which encloses a cavity in communication with a collar for connection to an ordinary syringe at an end wall opposite to outlet fittings for injection needles; the latter being distributed, as regards their number and arrangement, in accordance with a specific treatment for which each offtake pattern is intended.

US-A-3 595 231 discloses a device for a syringe for dividing a stream of the liquid to be injected into elementary stream feeding nipples. The device consists of a flat disc having a main flow nipple, connectable to a syringe, and a plurality of secondary flow nipples and a network of internal ducts for dividing the main stream of the liquid.

Lacking alternatives, this type of metal offtakes have gained wide spread acceptance even though their high cost makes it necessary to reuse each element for an indefinite number of applications, despite the fact that sterilisation, even where carefully carried out, may fail to ensure removal of residual substances from non-inspectable areas in the cavity.

The aim of the present invention is to obviate such serious deficiencies.

Within this aim, an object of the invention is also that of simplifying the syringe filling operations, because accurate metering of the various medicines to be mixed together currently requires the availability of a smaller syringe for drawing, from either vials or flacons, each component in the correct amount and then transferring it into the large syringe through a fitting in the offtakes or, after temporarily removing the plunger, directly into the barrel.

The syringe filling operation is made relatively easier if the medicines can be picked up from vials which are prepackaged with the required amounts, or if the graduation on the mixing barrel can be made sufficient for the amounts to be metered. In such cases, albeit infrequently occurring, it is sufficient to temporarily remove the offtake and connect to the syringe an ordinary injection needle.

The above mentioned aim and objects are achieved by a multi-needle syringe as described in claim 1.

Before turning to the description of the novel syringe, it would be appropriate to point out that, for each mesotherapeutical treatment, it is common practice to admix the medicines to a diluting liquid (physiologic solution or bi-distilled water) which is added in a significant proportion to bring the whole composition to an overall volume of 20 cm$^3$ of which only a small proportion is then injected. This explains why such a syringe may have features which render it uncapable of injecting all of the liquid contained therein.

The accompanying illustrative, and not limitative drawings consist of two sheets comprising the following figures:

Figures 1, 2 and 3 are side, top, and bottom views respectively, of a syringe having a barrel plunger and circular offtake for seven needles, both being complete with special caps;

Figure 4 is a longitudinal section AA through Figure 1;

Figure 5 and 6 are cross-sections BB and CC through Figure 4;

Figure 7 is a partly cutaway side view of a syringe with the barrel plunger shown during the filling step and the offtake outlet fittings (of the type having seven in-line needles) necessarily covered by the special air-tight caps;

Figure 8 is a cross-section DD through Figure 7;

Figure 9 shows views of the element to be slipped over each the two barrel grips for centering the cylindrical rod of the plunger.

As shown in drawing sheet 1, the syringe, which is formed from a suitable plastic material, comprises:

a graduated barrel 1 provided with grips 1A as with ordinary syringes but having a perforated end wall against which the plunger abuts and from which a small collar protrudes for the preferably pressure fitting of an offtake 2;

the offtake 2 is formed in two parts (2a and 2B) -which are joined together, preferably by ultrasonic welding, forming a distribution chamber therebetween, the interior of said distribution chamber defining a constrained siphon-like passage for each individual outlet fitting causing uniform distribution of the liquid flowing out of the distribution chamber through the set of holes formed in the part 2A similarly to those formed through the end wall of the barrel 1.

Furthermore, there are advantageously present:

air-tight caps 3 slipped over all of the outlet fittings of the offtake 2 to allow the syringe to be filled from the rear through the hollow plunger;

a cap 4 for attachment, after the syringe has been filled, to the outer end of the plunger in order to seal the outlet fitting in an air-tight fashion and provide adequate support for the hand pushing it in use;

a plunger 5 having a finned rod and an elastic seal 6, a conduit extending over its entire length from the head with the elastic seal 6 to the outer end fashioned as an inlet fitting, so that a needle can be attached to it which is then replaced with the special cap 4 once the filling drawing operation has been completed.

It has been stated in the foregoing that the multi-needle offtake is pressure fitted onto the collar of the syringe barrel, and this in order, as is the case with currently available means, for the mesotherapeutical operator, in treating the same patient, to be able to select and mount the most suitable offtakes for the various intervention areas.

It is not to be excluded, though, that the barrel and offtake may be welded together, preferably ultrasonically, to form a single body, and in such a case, the barrel could have a uniform cross-section and be without the perforated end wall, the function of which is mainly that of preventing accidental disengagement of the offtake owing to the plunger being pushed too far.

By so embodying multi-needle syringes, the added advantage would be secured of a reduction, by about 1 cm³, of the dead zone, i.e. of the non-injectable liquid. On the other hand, however, the operator would have limitations to his intervention and the whole syringe would have to be disposed of after each intervention, whereas with the interchangeable offtake system, the barrel and plunger, if made of a sterilisable material, could be resused for several interventions.

Further, by making the offtakes removable, the caps 3 of the individual outlet fittings could be replaced with a single air-tight cap, applied as the syringe is being filled, to the collar of the barrel 1 in lieu of the offtake 2.

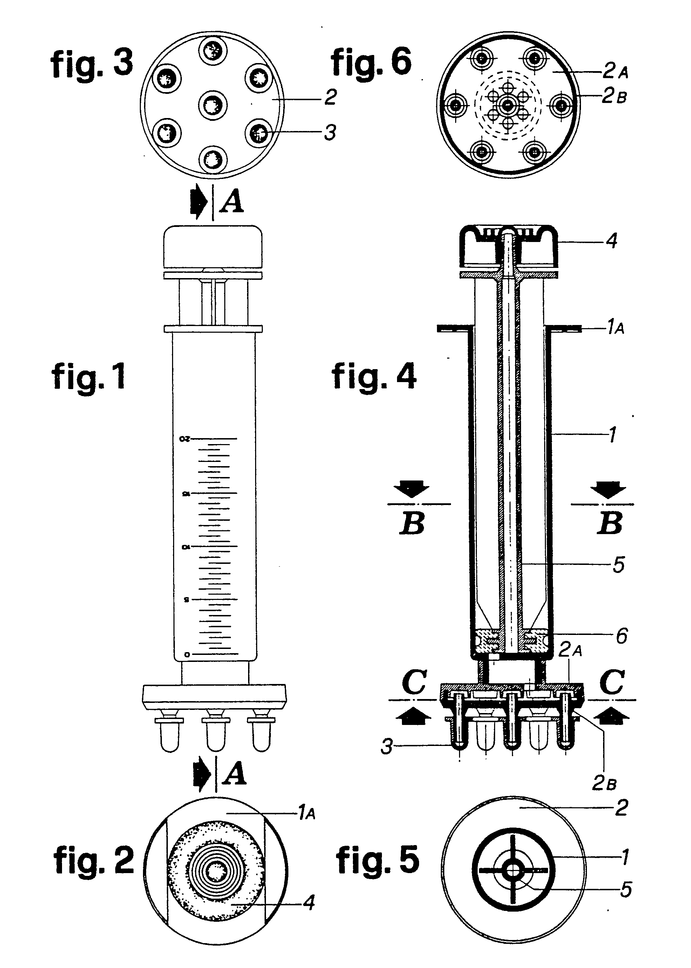

The syringe of drawing sheet 2 is shown during the filling step and with a needle 10 fitted in the inlet fitting of the plunger, whilst the syringe of drawing sheet 1 is alternatively provided with a different offtake from the previous one in that the needle outlet fittings are not rectilinearly aligned. However, this offtake is also configured internally with the siphon like outlet fittings as at 2 in Figure 4 and as in all the other offtakes which will be required for the various areas of mesotherapeutical intervention.

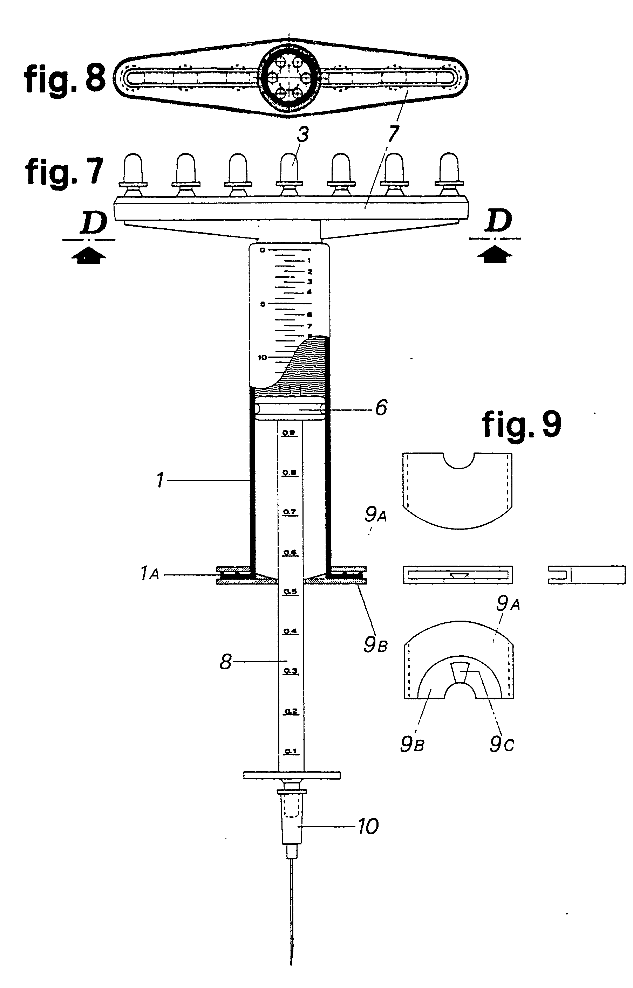

The plunger 8 is also different from that shown in drawing sheet 1, in that the former has four longitudinal web ribs which ensure constant centering in the barrel 1, and accordingly, a constantly effective tightness of the seal 6, whereas the latter, having a cylindrical rod, is held in a centered or coaxial trim with the barrel by virtue of a pair of elements 9 which, embodied as in the views of Figure 9 and section of Figure 7, clamp the grips 1a between two flat portions 9A and 9B, and the latter, in addition to restricting the barrel mouth is latched to it through a dog or elevation 9C formed thereon.

The variation with the cylindrical rod plunger 8 has been provided mainly to facilitate imprinting of an optional graduation because the conduit size is such as to enable metering of amounts so small that the mere graduation on the outer barrel 1 could not indicate with as much accuracy.

While the expedients described have been mainly devised to obviate some serious deficiencies of multi-needle syringes for mesotherapeutical use, this does not exclude a possible combination of the novel (hollow and graduated) plunger type with the barrels of ordinary syringes with a single needle, where to fill them, perfect metering of small amounts of liquid substances to be mixed is required.

## Claims

1. A multi-needle syringe particularly for mesotherapeutical applications, comprising a plunger (5) having one end provided with an elastic seal (6) slidable inside a graduated barrel (1) with the collar of which there is associated an offtake (2) having several outlet fittings (2B), characterised in that said plunger (5) has a suction conduit extending over its entire length for introducing into said barrel (1) during the drawing step liquid substances to be mixed, and in that the syringe comprises:
a cap (3) for each of the outlet fittings (2B) or for the collar of the barrel (1), the cap(s) being such as to be removably slipped over the fittings (2B) or the collar for sealing the fittings or the collar in an air tight fashion, and
a cap (4) for removable attachment to the end of the plunger remote from the seal (6) in order to seal the suction conduit of the plunger (5) in an air tight fashion,
so that the syringe can be filled through the hollow plunger (5) by drawing the plunger whilst the caps (3) are slipped over the outlet fittings (2B) or the collar and that the syringe can be emptied through the outlet fittings or collar by pushing the plunger whilst the cap (4) is attached to the plunger and the caps (3) are removed from the outlet fittings (2B) or collar.

2. A syringe according to Claim 1, characterised in that said plunger (5) has the end remote from said elastic seal (6) configured to accommodate an ordinary injection needle (10).

3. A syringe according to Claim 1 or 2, characterised in that said plunger (5, 8) has on its cylindrical surface a graduated scale for accurately metering said liquid substances to be mixed.

4. A syringe according to Claim 1, characterised in that it comprises at least one element (9a, 9b) removably associable with the mouth of said barrel (1) and having in one portion thereof at least one elevation (9c) for self-centering on said mouth by reducing the guiding cross-sectional area of said plunger (5—8).

5. A syringe according to Claim 1, characterised in that said barrel (1) has on its remote end from said mouth a perforated end cap.

6. A syringe according to Claim 5, characterised in that said end cap extends coaxially with a collar adapted to accommodate during the ejection step of said mixture a multi-needle offtake (7) and during the drawing step of said liquid substances said cap (3, 4).

7. A syringe according to Claim 1, characterised in that said multi-needle offtake (7) has at least one siphon-like costrained passage for each of said outlet fittings to even out the amount of said mixture in said outlet fittings.

## Patentansprüche

1. Mehrnadelspritze. insbesondere für mesotherapeutische Anwendungen, umfassend eine Kolbenanordnung (5). deren eines mit einer elastischen Dichtung (6) versehenes Ende im Inneren eines mit einer Skalenmarkierung versehenen Spritzenzylinders (1) gleitend angeordnet ist, wobei dem Zylinderhals ein Aufsatz (2) mit mehreren Auslaßstutzen (2B) zugeordnet ist, dadurch gekennzeichnet, daß die Kolbenanordnung (5) eine sich über ihre ganze Länge erstreckende Saugleitung aufweist, die dazu dient, während des Saughubes flüssige Substanzen, die miteinander vermischt werden sollen, in den Zylinder (1) einzufüllen, und daß die Spritze ferner umfaßt:

jeweils eine Kappe (3) für jeden der Auslaßstutzen (2B) bzw. für den Hals des Zylinders (1), wobei die Kappen so ausgebildet sind, daß sie abnehmbar über die Auslaßstutzen (2B) bzw. den Zylinderhals gestülpt werden können, um die Stutzen bzw. den Hals luftdicht zu verschließen, und

eine Kappe (4), die abnehmbar auf das der Dichtung (6) abgewandte Ende der Kolbenanordnung (5) aufsetzbar ist, um die Saugleitung der Kolbenanordnung (5) luftdicht zu verschließen,

so daß die Spritze durch die hohle Kolbenanordnung (5) durch Herausziehen der Kolbenanordnung bei auf die Auslaßstutzen (2B) bzw. den Zylinderhals gestülpten Kappen (3) gefüllt werden kann und daß die Spritze durch die Auslaßstutzen bzw. den Zylinderhals durch Drücken der Kolbenanordnung bei auf die Kolbenanordnung aufgesetzter Kappe (4) und von den Auslaßstutzen (2B) bzw. dem Zylinderhals abgenommenen Kappen (3) geleert werden kann.

2. Spritze nach Anspruch 1, dadurch gekennzeichnet, daß das von der elastischen Dichtung (6) abgewandte Ende der Kolbenanordnung (5) so ausgebildet ist, daß eine herkömmliche Injektionsnadel (10) aufsetzbar ist.

3. Spritze nach Anspruch 1 oder 2, dadurch gekennzeichnet, daß die Kolbenanordnung (5, 8) auf der Zylinderwand eine Skalenmarkierung zum genauen Abmessen der zu mischenden Flüssigsubstanzen aufweist.

4. Spritze nach Anspruch 1, dadurch gekennzeichnet, daß sie wenigstens ein Element (9a, 9b) umfaßt, welches abnehmbar mit der Mündung des Zylinders (1) verbindbar ist und welches in einem Bereich wenigstens einen Vorsprung (9c) zum Selbstzentrieren auf dieser Mündung durch Reduzierung der Führungsquerschnittsfläche für die Kolbenanordnung (5—8) hat.

5. Spritze nach Anspruch 1, dadurch gekennzeichnet, daß der Zylinder (1) an seinem der Mündung abgewandten Ende eine perforierte Abschlußkappe trägt.

6. Spritze nach Anspruch 5, dadurch gekennzeichnet, daß die Abschlußkappe koaxial zu einem Hals ausgerichtet ist, welcher dazu ausgelegt ist, beim Ausspritzen der Flüssigkeitsmischung einen Mehrnadelaufsatz (7) und beim Ansaugen der Flüssigsubstanzen die Kappen (3, 4) zu tragen.

7. Spritze nach Anspruch 1, dadurch gekennzeichnet, daß der Mehrnadelaufsatz (7) wenigstens einen siphonartigen, verengten Durchtritt für jeden der Auslaßstutzen hat, um die Menge der Flüssigkeit in den Auslaßstutzen einander anzugleichen.

**Revendications**

1. Seringue à aiguilles multiples particulièrement pour des applications mèsothèrapeutiques, comprenant un plongeur (5) ayant une extrémitè munie d'un joint èlastique (6) susceptible de coulisser à l'intèrieur d'un cylindre graduè (1) avec le collet duquel est associèe une voie d'ècoulement (2) ayant plusieurs raccords de sortie (2B), caractèrisèe en ce que ledit plongeur (5) prèsente un conduit d'aspiration s'ètendant sur toute sa longueur pour l'introduction dans ledit cylindre (1), durant l'ètape de prèlèvement, des substances liquides destinèes à être mèlangèes, et en ce que la seringue comprend:

un capuchon (3) pour chacun des raccords de sortie (2B) ou pour le collet du cylindre (1), le ou les capuchons ètant tels qu'ils coulissent de façon amovible sur les raccords (2B) ou le collet pour fermer avec ètanchèitè les raccords ou le collet d'une façon ètanche à l'air; et,

un capuchon (4) pour être liè de façon amovible à l'extrémité du plongeur èloignèe du joint (6), afin de fermer avec ètanchèitè le conduit d'aspiration du plongeur (5) d'une façon ètanche à l'air, de sorte que la seringue peut être remplie à travers le plongeur creux (5) en tirant le plongeur, tandis que les capuchons (3) sont agencés de façon coulissante sur les raccords de sortie (2B) ou le collet, et que la seringue peut être vidèe à travers les raccords de sortie ou le collet en poussant le plongeur tandis que le capuchon (4) est liè au plongeur et que les capuchons (3) sont retirès des raccords de sortie (2B) ou du collet.

2. Seringue selon la revendication 1, caractèrisèe en ce que l'extrémitè du plongeur (5), èloignèe dudit joint èlastique (6), est conformèe pour recevoir une aiguille d'injection usuelle (10).

3. Seringue selon la revendication 1 ou 2, caractèrisèe en ce que ledit plongeur (5, 8) prèsente sur sa surface cylindrique une èchelle graduèe pour doser de façon prècise lesdites substances liquides destinèes à être mèlangèes.

4. Seringue selon la revendication 1, caractèrisèe en ce qu'elle comprend au moins un èlément (9a, 9b) susceptible d'être associè, de façon amovible, à l'embouchure dudit cylindre (1) et ayant dans une portion de celui-ci au moins une saillie (9c) pour l'autocentrage sur ladite embouchure en rèduisant la section transversale de guidage dudit plongeur (5—8).

5. Seringue selon la revendication 1, caractèrisèe en ce que ledit cylindre (1) prèsente sur son extrémitè èloignèe de ladite embouchure un capuchon d'extrémitè perforè.

6. Seringue selon la revendication 5, caractèrisèe en ce que ledit capuchon d'extrémitè s'ètend coaxialement à un collet adaptè pour recevoir, durant l'ètape d'èjection dudit mèlange, une voie d'ècoulement (7) à aiguilles multiples et, durant l'ètape de prèlèvement desdites substances liquides, ledit capuchon (3, 4).

7. Seringue selon la revendication 1, caractèrisèe en ce que ladite voie à aiguilles multiples (7) prèsente au moins un passage forcè du type syphon pour chacun desdits raccords de sortie pour répartir la quantitè dudit mèlange dans lesdits raccords de sortie.

fig. 3

fig. 6

2

3

2A

2B

fig. 1

fig. 4

4

1A

1

B

B

5

6

2A

C

C

3

2B

A

fig. 2

fig. 5

1A

4

2

1

5

1

EP 0 167 662 B1

fig. 8

fig. 7

3

7

D          D

fig. 9

6

1

9A

1A

9B

8

9A

9B          9C

10

2